Europäisches Patentamt

European Patent Office

Office européen des brevets

(11) Publication number: **0 329 214**
A1

## EUROPEAN PATENT APPLICATION

(21) Application number: 89200209.8

(51) Int. Cl.⁴: **C07C 126/02** , **C07C 126/08**

(22) Date of filing: 01.02.89

(30) Priority: 04.02.88 NL 8800259

(43) Date of publication of application:
23.08.89 Bulletin 89/34

(84) Designated Contracting States:
ES FR GB GR IT

(71) Applicant: **STAMICARBON B.V.**
**Mijnweg 1**
**NL-6167 AC Geleen(NL)**

(72) Inventor: **Jonckers, Kees**
**Illikhoven 13**
**NL-6116 AK Susteren(NL)**

(54) **Process for concentrating a urea solution.**

(57) Process for concentrating a urea solution by
evaporation of water from the urea solution, in which
first
- in a synthesis zone a urea synthesis solution
containing carbamate and free ammonia is formed
from carbon dioxide and an excess of ammonia,
- in a first decomposition step, at synthesis pressure
or lower pressure, a portion of the carbamate is
decomposed,
- in a further decomposition step, at a pressure
between 4 and 40 bar, a further portion of the
carbamate still present is decomposed,
- the urea solution eventually obtained is concen-
trated by evaporation in at least two steps, in the
first step use being made of the heat released upon
condensation of the gas mixture obtained in the
decomposition step operated at between 4 and 40
bar.

The pressure in the decomposition step oper-
ated at between 4 and 40 bar is set at the minimum
value at which, with virtually complete condensation
of the gas mixture, the amount of heat released is
sufficient for reaching the desired final concentration
and temperature of the urea solution in the first
evaporation step.

FIG 2

## PROCESS FOR CONCENTRATING A UREA SOLUTION

The invention relates to a process for concentrating a urea solution by evaporation of water from the urea solution.

In the preparation of urea from carbon dioxide and an excess of ammonia, in the synthesis zone first a urea synthesis solution containing carbamate and free ammonia is formed at a pressure of 125-350 bar and the corresponding temperature of 170-250¤C. The carbamate is decomposed into ammonia and carbon dioxide, and the decomposition products, together with a portion of the ammonia and water present in the solution, are removed from the solution in a number of steps. Eventually a urea solution in water is obtained which contains about 70-75 wt.% urea and in which ammonia and carbon dioxide are still present. This solution is not suitable for fertilizer purposes or for use in resin manufacture. As a rule, it must first be processed into solid urea. In doing so the water, about 25-30 wt.%, and the ammonia and carbon dioxide still present are removed at least for the major part by evaporation, or urea is allowed to crystallize out from the solution and the crystals are melted, after which the resulting urea melt is converted into granulate. Since exposure of urea solutions to high temperatures gives rise to decomposition of urea and formation of biuret, which biuret is undesirable both when urea is used for fertilizer purposes and for resin manufacture, as a rule evaporation of urea solutions takes place under reduced pressure so as to avoid excessively high evaporation temperatures. In addition, for economic reasons the evaporation process is mostly carried out in two or more steps, most of the water present being removed under a moderately reduced pressure in the first step(s), following which evaporation is continued in the last step under a much more strongly reduced pressure until a virtually water-free urea melt, containing less than 0.5 wt.% water, is obtained. However, it also is possible to process the solu tion obtained in the first evaporation step, which usually has a urea concentration of 90-97 wt.%, without further concentrating, for instance in a granulation process.

In practice for evaporation use is mostly made of evaporators in which the solution to be concentrated is passed through the tubes of a vertical tube bundle and the heat needed for evaporation is obtained by condensation of low-pressure steam in the shell space provided around the tube bundle.

It has already been suggested to utilize the heat, that can be generated from gaseous and liquid process streams obtained during urea synthesis, for concentrating urea solutions, see for instance the non-prepublished Dutch patent application 8602770. The process described in said patent application relates to a urea synthesis in which, in a synthesis zone at a pressure of between 125 and 350 bar, first a urea synthesis solution is formed, which contains carbamate and free ammonia besides urea and water, this urea synthesis solution is treated in a number of decomposition steps, including a first step at synthesis pressure or lower pressure and a second step at 4-40 bar, to decompose carbamate and remove the decomposition products, together with an amount of water and the free ammonia present, from the solution, and finally the remaining solution is concentrated in two evaporation steps. The heat needed in the first evaporation step is obtained by condensation of the gas mixture obtained in the second decomposition step at a pressure of 4-40 bar, which gas mixture has been formed upon expansion of the stripped urea synthesis solution and the consecutive heat exchange of the expanded reaction mixture with the gas mixture from the first decomposition step that is being condensed to yield carbamate.

When the heat released upon condensation is not sufficient to cover the heat requirements of this evaporation step, then an additional amount of heat is supplied by supplementary condensation of low-pressure steam.

In effecting the urea synthesis, for trouble-free operation and for obtaining a good final product it is desirable for the temperature and the concentration of the urea solution discharged from the first evaporation stage to be kept at predetermined constant values, for instance a concentration of 95 wt.% and a temperature of 130¤C. As a rule fluctuations in the concentration and/or temperature of the urea solution in the evaporation step can be corrected by adjusting the heat supply to this evaporation step. If, in evaporating a urea solution using heat obtained by condensation of an $NH_3$-, $CO_2$- and $H_2O$-containing gas mixture, supplemented by condensation of low-pressure steam, as suggested in the above-mentioned Dutch patent application 8602770, the amount of steam is restricted to a minimum, then corrections in the heat supply will have to be effected by adjustment of the amount of gas mixture to be condensed. In practice this means that the steam pressure of the steam fed to the stripping zone will have to be varied. The disadvantage of such a mode of action is that disturbances in the evaporation step have repercussions on process operation in the high-pressure part of the urea synthesis, resulting in unsteady plant operation.

The object of the invention is to realize a process for the preparation of urea in which the heat requirements of the first evaporation step are economically met by condensation of an $NH_3$-, $CO_2$-and $H_2O$-containing gas mixture, without supplementary steam heating being required. This is achieved by generation of only so much carbamate dissociation gases and virtually complete condensation thereof, at such a pressure that the condensation temperature always remains higher than the temperature of the urea solution to be evaporated, that enough heat is supplied to reach a predetermined final concentration and final temperature. If water or an aqueous carbamate solution is also supplied at an optimum place during condensation, it is achieved that the pressure can be kept at a minimum value.

The invention therefore relates to a process for concentrating a urea solution by evaporation of water from the urea solution, in which first
- in a synthesis zone, at a pressure of 125-350 bar and the corresponding temperature of 170-250¤C, a urea synthesis solution containing carbamate and free ammonia is formed from carbon dioxide and an excess of ammonia,
- in a first decomposition step, at synthesis pressure or lower pressure and while supplying heat, a portion of the carbamate is decomposed and the resulting gas mixture is condensed at least in part in a first condensation zone and the condensate and any non-condensed portion is returned to the synthesis zone,
- in one or more further decomposition steps a further portion of the carbamate still present is decomposed and the gas mixture formed is separated, in the first of the further decomposition steps a pressure of 4-40 bar being maintained and heat being supplied,
- the remaining urea-containing solution is concentrated by evaporation in at least two steps, in the first step use being made of the heat released upon condensation of the gas mixture obtained in the first of the further decomposition steps.
The invention is characterized in that the pressure in the first of the further decomposition steps is set at the minimum value at which, with virtually complete condensation of the gas mixture, the amount of heat released is sufficient for reaching the desired final concentration and temperature of the urea solution in the first evaporation step.

Preferably, it will be desired to discharge the urea solution from the first evaporation step with a concentration of 94-96 wt.% and a temperature of 129-131¤C, since a solution having such a concentration can without problems either be evaporated further under an increased vacuum for conversion into a practically water-free melt, or as such be subjected directly to a granulation process. The pressure in the first of the further decomposition steps will usually be chosen between 15 and 22 bar and will preferably be approximately 18 bar, since then a sufficiently large amount of heat of a sufficiently high temperature level will be released upon condensation of the gas mixture formed. Since it is desirable for the heat of condensation to become available at the highest possible temperature level, during condensation of the gas mixture the pressure will as far as possible be kept equal to the pressure in the first of the further decomposition steps.

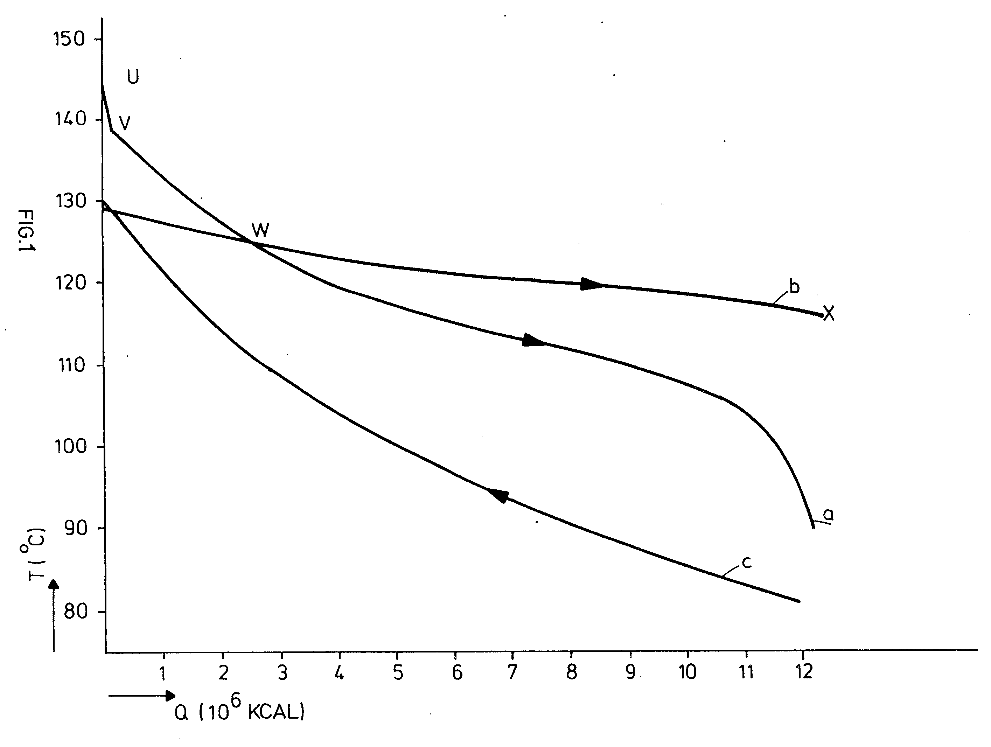

To ensure that the temperature of the condensing gas mixture is always higher than that of the urea solution to be concentrated, the heat exchange between the solution to be concentrated and the condensing gas mixture is to be effected countercurrently. This becomes clear on the basis of Figure 1, representing a Q-t diagram (Q = amount of heat transferred to urea solution to be concentrated; t = temperature of condensing gas mixture).

In the figure, line a represents the condensation line of the gas mixture in the absence of water, line b is the condensation line in the presence of water, and line c the evaporation line of the urea solution to be concentrated. When the aqueous solution is fed to the condensing gas mixture at a place where the temperature substantially corresponds with that of point W, then condensation proceeds according to the line UVWX, and the temperature difference between the urea solution to be evaporated and the condensing gas mixture always is sufficient to guarantee good heat transfer.

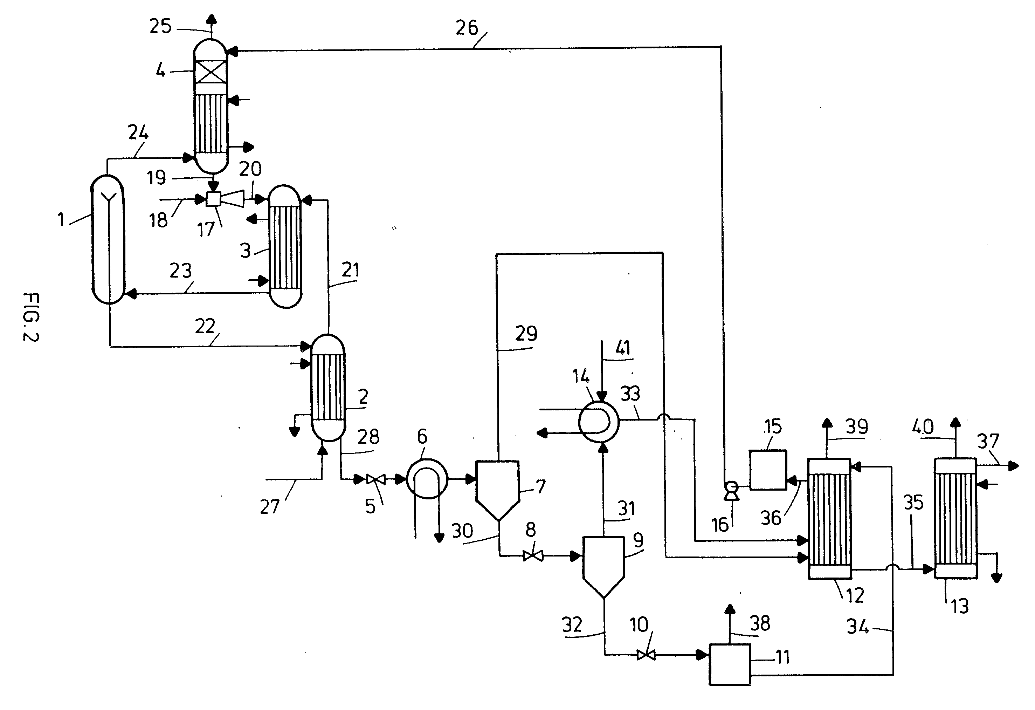

The invention will be elucidated with reference to Figure 2 and the example, however, without being restricted thereto.

In the mode of realization according to Figure 2, 1 represents a synthesis zone, 2 a first decomposition step, for instance a stripping zone, 3 a first condensation zone, and 4 a washing column. This equipment forms part of the high-pressure part of the urea synthesis, which can be operated at a pressure of 125-350 bar. 5, 8 and 10 denote expansion valves, 6 a heating zone, 7 and 9 represent gas-liquid separators, 11 is a storage vessel for the urea solution to be evaporated. 12 and 13 denote, respectively, the first and the second evaporation step, 14 represents a second condensation zone and 15 a storage vessel for the carbamate solution obtained upon the heat exchange in the first evaporation step. 16 denotes a pump for pumping carbamate solution and 17 a liquid ejector.

Liquid ammonia is passed via 18, ejector 17, and, together with the carbamate solution supplied via 19 from washing column 4, via 20 into first condensation zone 3. Via 21 this zone is also fed with the gas mixture from stripping zone 2. This gas mixture is obtained by passing the urea synthesis solution from synthesis zone 1 into stripping zone 2 while supplying heat, the solution flowing counter-current to carbon dioxide introduced into this zone via 27. This first condensation zone may, for instance, be designed as a vertical tubular heat exchanger. The heat released in this zone upon ammonium carbamate formation is discharged by means of boiler feed water, which is thereby converted into low-pressure steam of 4-5 bar. The stripping zone, too, may be designed as a vertical tubular heat exchanger. The heat required for stripping is supplied in the form of high-pressure steam of, for instance, 15-30 bar. The carbamate solution formed in first condensation zone 3 and the non-condensed gas are fed to synthesis zone 1 via 23. In this zone, the further condensation of ammonia and carbon dioxide, yielding carbamate, produces enough heat to cover the heat requirements of the endothermic conversion of carbamate into urea. The gas mixture not condensed in the synthesis zone, which mixture contains the inert gases introduced into the process with the fresh ammonia and carbon dioxide, and optionally as passivation air or oxygen, is supplied via 24 to washing column 4, where the ammonia and carbon dioxide present in the gas are scrubbed out with the aid of carbamate solution supplied via 26. The inert gases are vented via 25.

The stripped urea solution is discharged from stripping zone 2 and passed to gas-liquid separator 7 via 28, expansion valve 5, where the pressure is reduced to 4-40 bar, for instance approximately 18 bar, and via heating zone 6, where the reaction mixture is heated to 130-180°C, for instance approximately 165°C, by heat exchange, which results in decomposition of a portion of the carbamate still present. From the separator the gas phase formed, a mixture of mainly ammonia, carbon dioxide and water vapour, is discharged via 29 and the remaining liquid phase via 30. By means of expansion valve 8 the pressure of the liquid phase is reduced to 1-10 bar, for instance approximately 7 bar, and the resulting gas-liquid mixture is passed to gas-liquid separator 9. The gas phase obtained here, a mixture of mainly ammonia, carbon dioxide and water vapour, is passed via 31 to second condensation zone 14, where it is condensed to a carbamate solution, for instance by means of process condensate supplied via 41. The heat released in condensation is discharged by means of cooling water. The liquid phase obtained in gas-liquid separator 9 flows via 32 and expansion valve 10, where the solution is expanded to atmospheric or lower pressure, for instance approximately 0.6 bar, to storage vessel 11. From this vessel the solution obtained, containing for instance about 70 wt.% urea, and also still containing ammonia and carbon dioxide, is passed to first evaporation step 12. Subsequently, the urea solution, for instance already concentrated to more than 90 wt.% urea, is passed to second evaporation step 13 via 35. The heating zones of these evaporation steps may, for instance, be designed as vertical tubular heat exchangers. The water vapour obtained in both evaporation steps, containing small amounts of liquid urea, ammonia and carbon dioxide, is sent, via 39 and 40, respectively, after condensation, to a unit, not shown, for process condensate upgrading, as is the gas phase discharged from storage vessel 11 via 38.

The heat needed for evaporation in second evaporation step 13 is obtained by condensation of low-pressure steam. The heat required in the first evaporation step is obtained by condensation of the gas mixture discharged from gas-liquid separator 7 via 29. To this end, this gas mixture is passed to the shell side of the tubular heat exchanger of first evaporation step 12, to which simultaneously via 33 the carbamate solution from second condensation zone 14 is supplied. As a result, the dew point of the gas mixture to be condensed is raised. The carbamate solution also serves as condensation and dissolution medium for the gas mixture to be condensed. The gas-liquid mixture is passed through the heating zone countercurrent to the urea solution to be evaporated. The carbamate solution formed upon condensation in the shell side is passed via 36 to storage vessel 15, brought to synthesis pressure by pump 16 and passed, via 26, to the upper part of washing column 4 for removal of ammonia and carbon dioxide from the gas mixture containing the inert gases, which has been discharged from synthesis zone 1 via 24.

The evaporated urea solution is discharged via 37.

Example

Using the process as described, urea was prepared according to the mode of realization as represented in Figure 2 in a plant having a production capacity of 1500 tonnes per day. The amounts are given in kg per hour. The pressure employed in the high-pressure part of the plant was 137.3 bar. The temperature in the reaction zone was 183°C. The condensation zone was supplied with 35,660 kg $NH_3$, which contained 107 kg $H_2O$ and had been preheated to 77°C, and 68,892 kg of a carbamate solution containing 28,373 kg $CO_2$, 26,995 kg $NH_3$, 13,450 kg $H_2O$ and 24 kg urea. The stripping zone was fed with 193,678 kg urea synthesis solution and stripping was effected using 47,906 kg gas mixture that contained 45,862 kg $CO_2$ and for the remainder consisted of inerts, mainly air. From the stripping zone a solution was obtained that contained 63,525 kg urea, 208 kg biuret, 19,730 kg $CO_2$, 17,741 kg $NH_3$, 32,342 kg $H_2O$ and 19 kg inert components. The pressure of the stripped urea synthesis solution was reduced while heat was being supplied and the remaining solution was further upgraded to an approximately 75 wt.% urea solution. This solution contained 63,525 kg urea, 208 kg biuret, 34 kg $CO_2$, 400 kg $NH_3$ and 22,272 kg water. The temperature of the solution was 95°C. The solution was passed into the top section of the tubes of a tubular heat exchanger at a pressure of 0.33 bar. The shell space of the heat exchanger was divided into ten compartments by means of horizontal baffles that were provided with openings. The gas mixture, obtained when the pressure of the stripped urea synthesis solution was reduced while heat was being supplied, was introduced into the first compartment down in the shell space of the heat exchanger. A carbamate solution, containing 24 kg urea, 5,717 kg $CO_2$, 8,299 kg $NH_3$ and 8,972 kg water and having a temperature of 46°C, was passed into the third compartment of the shell space. To concentrate the urea solution to a 95 wt.% solution of 130°C, 11,685 x $10^6$ kcal were needed. This amount was obtained by expanding the stripped urea synthesis solution to a pressure of 17.9 bar and condensing the gas mixture, in total 31,159 kg having a temperature of 158°C, consisting of 16,320 kg $CO_2$, 10,881 kg $NH_3$, 3,939 kg water and the remainder inerts, for which condensing operation use was made of the carbamate solution. The shell space pressure was controlled on the basis of the final concentration and pressure of the urea solution to be discharged. From the top of the last compartment of the shell space a carbamate solution containing 20,895 kg $CO_2$, 18,413 kg $NH_3$, 12,840 kg water and 24 kg urea was discharged as well as a gas mixture containing, besides the inert gases, 1,142 kg $CO_2$, 768 kg $NH_3$ and 14 kg water. From the bottom of the heat exchanger a urea solution was obtained which had a temperature of 130°C and contained 62,980 kg urea, 263 kg biuret, 3 kg $NH_3$ and 3,336 kg water.

## Claims

1. Process for concentrating a urea solution by evaporation of water from the urea solution, in which first
- in a synthesis zone at a pressure of 125-350 bar and the corresponding temperature of 170-250°C a urea synthesis solution containing carbamate and free ammonia is formed from carbon dioxide and an excess of ammonia,
- in a first decomposition step, at synthesis pressure or lower pressure and while supplying heat, a portion of the carbamate is decomposed and the resulting gas mixture is condensed at least in part in a first condensation zone and the condensate and any non-condensed portion is returned to the synthesis zone,
- in one or more further decomposition steps a further portion of the carbamate still present is decomposed and the resulting gas mixture is separated, in the first of the further decomposition steps a pressure of 4-40 bar being maintained and heat being supplied,
- the remaining urea-containing solution is concentrated by evaporation in at least two steps, in the first step use being made of the heat released upon condensation of the gas mixture obtained in the first of the further decomposition steps, characterized in that the pressure in the first of the further decomposition steps is set at the minimum value at which, with virtually complete condensation of the gas mixture, the amount of heat released is sufficient for reaching the desired final concentration and temperature of the urea solution in the first evaporation step.

2. Process according to claim 1, characterized in that the urea solution is discharged from the first evaporation step with a concentration of 94-96 wt.% and a temperature of 129-131°C.

3. Process according to either of claims 1 and 2, characterized in that the pressure in the first of the further decomposition steps is chosen within the range of 15-22 bar, preferably at approximately 18 bar.

4. Process for concentrating a urea solution as described and elucidated with reference to the figures and the example.

5. Concentrated urea solution obtained by the process according to one or more of claims 1-3.

FIG.1

FIG. 2

## PARTIAL EUROPEAN SEARCH REPORT

European Patent Office

which under Rule 45 of the European Patent Convention shall be considered, for the purposes of subsequent proceedings, as the European search report

Application number

EP 89 20 0209

## DOCUMENTS CONSIDERED TO BE RELEVANT

| Category | Citation of document with indication, where appropriate, of relevant passages | Relevant to claim | CLASSIFICATION OF THE APPLICATION (Int. Cl.⁴) |
|---|---|---|---|
| P,X | EP-A-0 266 840 (STAMICARBON)<br>* Claims *<br>& NL-A-86 02 770 (Cat. D)<br>-- | 1,5 | C 07 C 126/02<br>C 07 C 126/08 |
| X | EP-A-0 145 054 (UNIE VAN KUNSTMEST-FABRIEKEN)<br>* Whole document *<br>-- | 1-3,5 | |
| X | EP-A-0 093 466 (UNIE VAN KUNSTMEST-FABRIEKEN)<br>* Whole document *<br>-- | 1-3,5 | |
| A | EP-A-0 212 744 (STAMICARBON)<br>* Whole document *<br>-- | 1-3,5 | |
| A | EP-A-0 213 669 (STAMICARBON)<br>* Whole document *<br>--------- | 1-3,5 | TECHNICAL FIELDS SEARCHED (Int. Cl.⁴)<br><br>C 07 C 126/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-3,5

Claims searched incompletely:

Claims not searched: 4

Reason for the limitation of the search:

Rule 29.6 of the European Patent Convention

| Place of search | Date of completion of the search | Examiner |
|---|---|---|
| The Hague | 21-04-1989 | VAN GEYT |

EPO Form 1505.1 03.82